# EUROPÄISCHE PATENTSCHRIFT

(11) **EP 1 833 813 B1**
(45) Veröffentlichungstag und Bekanntmachung des Hinweises auf die Patenterteilung: **26.05.2010**
(21) Anmeldenummer: 05821911.4
(22) Anmeldetag: 14.12.2005
(51) Int. Cl.: C07D 333/34

(54) **VERFAHREN ZUR HERSTELLUNG VON SUBSTITUIERTEN THIOPHENSULFONYLISOCYANATEN**
METHOD FOR THE PRODUCTION OF SUBSTITUTED THIOPHENESULFONYL ISOCYANATES
PROCEDE POUR PRODUIRE DES THIOPHENESULFONYLISOCYANATES SUBSTITUES

(30) Priorität: 29.12.2004 DE 102004063192
(43) Veröffentlichungstag der Anmeldung: 19.09.2007
(73) Patentinhaber: Bayer CropScience Aktiengesellschaft, 40789 Monheim (DE)
(72) Erfinder: GELLER, Thomas, 51519 Odenthal (DE); STÖLTING, Jörn, 51061 Köln (DE)
(86) Internationale Anmeldenummer: PCT/EP2005/013407
(87) Internationale Veröffentlichungsnummer: WO 2006/072376

(56) Entgegenhaltungen:
- US-A- 4 481 029

## Beschreibung

Die Erfindung betrifft ein neues Verfahren zur Herstellung von 2,4-disubstituierten Thiophen-3-sulfonylisocyanaten, welche als Zwischenprodukte für Wirkstoffe in der Landwirtschaft, insbesondere für substituierte, herbizid aktive Thienylaminocarbonyltriazolinone (vgl. WO 01/05788) und Sulfonylharnstoffe (WO 01/10863), beschrieben sind.

Es ist bekannt, dass bestimmte substituierte Thienylsulfonylisocyanate durch Phosgenierung in Gegenwart von organischen Basen, insbesondere der Base DABCO, aus den entsprechenden Sulfonamiden hergestellt werden können (EP-A 30 142, US-A 4,481,029). Nachteilig bei diesen Verfahren ist jedoch, dass eine teure organische Base eingesetzt werden muss.

Erfindungsgemäß wurde nun gefunden, dass die Herstellung der speziellen substituierten 2-Alkyl-4-Alkoxycarbonyl-disubstituierten Thiophen-3-sulfonylisocyanate in hoher Ausbeute ohne nennenswerte Bildung von Nebenprodukten ebenfalls durchführbar ist, ohne die üblichen Basen zuzusetzen.

Es wurde demnach gefunden, dass man Verbindungen der allgemeinen Formel (I) 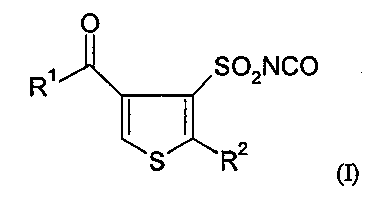 in welcher
- R¹: für Alkoxy mit 1 bis 4 Kohlenstoffatomen steht, und
- R²: für Alkyl mit 1 bis 4 Kohlenstoffatomen steht,
in sehr guten Ausbeuten und in hoher Reinheit erhält,
wenn man Verbindungen der Formel (II) 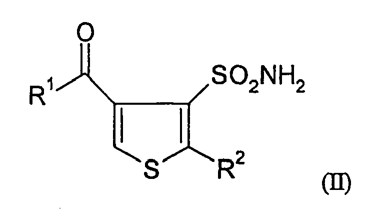 in welcher R¹ und R² die oben angegebene Bedeutung haben,
mit Phosgen in Abwesenheit einer Base und in Gegenwart eines oder mehrerer Verdünnungsmittel sowie gegebenenfalls eines Katalysators umsetzt.

In der Formel (I) steht R¹ bevorzugt für Methoxy, Ethoxy, n- oder i-Propoxy. R² steht bevorzugt für Methyl, Ethyl, n- oder i-Propyl. R¹ steht besonders bevorzugt für Methoxy. R² steht besonders bevorzugt für Methyl.

Durch die Umsetzung der Sulfonamide ohne Verwendung einer organischen Base, wurde überraschend ein neues Verfahren gefunden, welches die Herstellung der 2,4-disubstituierten Thienyl-3-sulfonylisocyanate in sehr guten Ausbeuten und in hoher Reinheit bei kurzer Reaktionszeit erlaubt.

Das erfindungsgemäße Verfahren stellt somit eine Bereicherung des Standes der Technik dar, da es eine sehr vorteilhafte Herstellung von 2,4-disubstituierten Thienyl-3-sulfonylisocyanaten erlaubt. Damit wird der Zugang zu den auf diesen Zwischenprodukten basierenden herbiziden Thienylaminocarbonyltriazolinonen und Sulfonylharnstoffen erleichtert.

Die Verbindungen der Formel (II) sind bekannt und können wie in der WO 01/05788 angegeben oder nach prinzipiell bekannten Verfahren hergestellt werden (vgl. z.B. EP-A 30 412, US-A 4,481,029).

Bei der erfindungsgemäßen Umsetzung der substituierten Sulfonamide mit Phosgen arbeitet man im Allgemeinen bei Temperaturen zwischen 20°C und 170°C, vorzugsweise zwischen 100°C und 150°C, besonders bevorzugt 125°C und 135°C. Als Verdünnungsmittel bei der Umsetzung werden bevorzugt gegebenenfalls halogenierte aromatische Kohlenwasserstoffe eingesetzt, z.B. Chlorbenzol, Toluol oder Xylol. Xylol und Chlorbenzol sind als Verdünnungsmittel besonders bevorzugt.

Bei der erfindungsgemäßen Umsetzung der Sulfonamide mit Phosgen betragen die Reaktionszeiten im Allgemeinen zwischen 30 Minuten und 6 Stunden, vorzugsweise zwischen 2 und 4 Stunden.

Zur Durchführung des erfindungsgemäßen Verfahrens setzt man zur Herstellung der Verbindungen der Formel (I) bevorzugt das Reagenz äquimolar oder im Überschuss ein. Im Allgemeinen werden je Mol Sulfonamid der Formel (II) zwischen 1,0 und 10 Mol, vorzugsweise zwischen 1,0 und 3 Mol Phosgen eingesetzt. Aber auch größere Überschüsse an Phosgen können erfindungsgemäß verwendet werden.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Das erfindungsgemäße Verfahren sollte vorteilhafterweise in Gegenwart eines Katalysators durchgeführt werden. Als Katalysator kann eine katalytisch wirkende Menge eines Butylisocyanats bzw. Pentylisocyanats verwendet werden. Die bevorzugte Konzentration an diesem Isocyanat bei der Reaktion beträgt 0,1 bis 5 Mol je Mol Sulfonamid, vorzugsweise 0,7 bis 1,3 Mol.

Naturgemäß lassen sich die substituierten Sulfonamide auch mit dem als Katalysator verwendeten Isocyanat zu den entsprechenden Harnstoffen umsetzen. Diese können dann in einem zweiten Schritt mit Phosgen ebenfalls in die entsprechenden Isocyanate umgesetzt werden.

Die nach dem erfindungsgemäßen Verfahren erhaltenen Verbindungen der Formel (I) können vor ihrer Verwendung zur Herstellung von herbiziden Endprodukten isoliert werden. Es ist aber auch möglich die erhaltenen Verbindungen unmittelbar ohne Zwischenisolierung weiter umzusetzen. Es ist sogar bevorzugt, zur Herstellung von herbiziden Thienylsulfonylaminocarbonyltriazolinonen die erhaltenen Verbindungen der Formel (I) unmittelbar mit den Aminen (z.B. den Triazolinonen) umzusetzen.

Erfindungsgemäß ist daher auch ein Verfahren bei dem die Verbindungen der Formel (I) in einem nachfolgenden Reaktionsschritt ohne Zwischenisolierung mit einem Triazolinon umgesetzt werden ("Eintopfverfahren", vgl. Herstellungsbeispiel 3).

### Herstellungsbeispiele:

### Beispiel 1

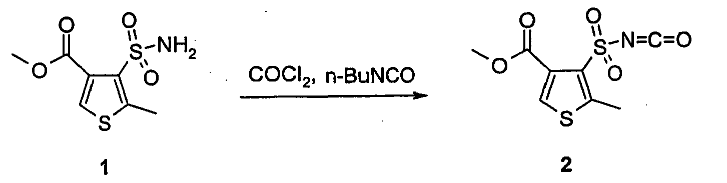

23,5 g Methyl 4-(aminosulfonyl)-5-methylthiophene-3-carboxylat (1) (100 mmol) wurden in 250 ml Xylol vorgelegt. Von dieser Mischung wurden am Wasserabscheider ca. 70 ml Xylol abdestilliert. Nach Zusatz von 9,9 g n-Butylisocyanat (100 mmol) wurde die Reaktionsmischung auf Rückfluß erwärmt. Über einen Zeitraum von 3 h wurden anschließend 24 g Phosgen so eingeleitet, dass die Temperatur bei ca. 130 °C blieb (überschüssiges Phosgen wurde durch einen Phosgenkühler im System gehalten). Zur Aufarbeitung wurde der Ansatz auf Raumtemperatur gekühlt und überschüssiges Phosgen durch Durchleiten eines Argonstroms entfernt. Anschließend erfolgte eine destillative Aufreinigung, bei der das Butylisocyanat zurückgewonnen werden kann. Das Methyl-4-(isocyanatosulfonyl)-5-methylthiophene-3-carboxylat (2) kann dann nach einer Destillation unter vermindertem Druck als farbloser Feststoff gewonnen werden. Man erhielt 22 g des Methyl 4-(isocyanatosulfonyl)-5-methylthiophene-3-carboxylats (2) (83 % d. Th., Gehalt: 98 %, Kp.: 120 °C, 0,4 mbar). Der Identitätsnachweis und die Gehaltsbestimmung wurden über Derivatisierung mit Methanol zum entsprechenden Carbamat geführt (¹H NMR (400 MHz, d⁶DMSO): 2,72 (s), 3,61 (s), 3,79 (s), 8,00 (s), 11,96 (s)).

### Beispiel 2

Unter den oben genannten Bedingungen wurden 23,5 g Methyl 4-(aminosulfonyl)-5-methylthiophene-3-carboxylat (1) mit 11,3 g Pentylisocyanat (100 mmol) in Gegenwart von Phosgen zum Methyl 4-(isocyanatosulfonyl)-5-methylthiophene-3-carboxylat (2) umgesetzt. Man erhielt ebenfalls 22 g dieses Isocyanats (83 % d. Th., Gehalt: 99 %, Kp.: 120 °C, 0,4 mbar). Der Identitätsnachweis und die Gehaltsbestimmung wurden über Derivatisierung mit Methanol zum entsprechenden Carbamat gerührt (¹H NMR (400 MHz, d⁶DMSO): 2,72 (s), 3,61 (s), 3,79 (s), 8,00 (s), 11,96 (s)).

### Beispiel 3

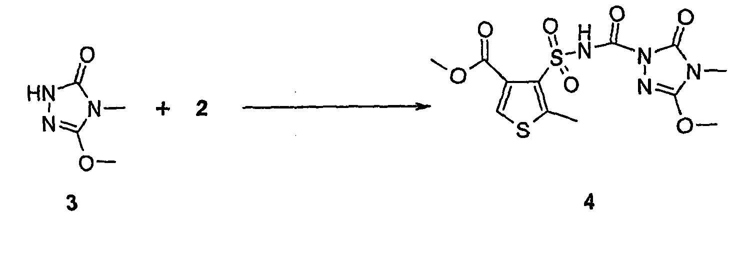

12,9 g 5-Methoxy-4-methyl-2,4-dihydro-3H-1,2,4-triazol-3-one (3) (100 mmol) wurden in 200 ml Xylol vorgelegt und auf auf 70 °C erwärmt. Bei dieser Temperatur wurden 119 g einer
22 %igen Lösung des Methyl 4-(isocyanatosulfonyl)-5-methylthiophene-3-carboxylats (2) (100 mmol) in trockenem Xylol zudosiert. Die Mischung wurde für 5,8 h bei 70 °C nachgerührt. Anschließend erfolgte bei Raumtemperatur eine Aufarbeitung durch Filtration des ausgefallenen Produkts. Man erhielt nach einer Wäsche mit 50 ml Xylol und anschließender Trocknung 36,4 g Methyl 4-({[(3-methoxy-4-methyl-5-oxo-4,5-dihydro-1H-1,2,4-triazol-1-yl)carbonyl]amino}sulfonyl)-5-methylthiophene-3-carboxylat (4) (91 % d. Th. Gehalt gegen Standard: 97,5 %) vom Schmelzpunkt 201 °C.

Alternativ zur Zweischrittprozedur (separate Herstellung des Isocyanats und anschließende Umsetzung mit dem Amin) kann nach dem Entfernen des Phosgens (Isocyanat-Herstellung) und Abdestillieren des als Katalysator eingesetzten Isocyanats, die aus der Herstellung von Methyl 4-(isocyanatosulfonyl)-5-methylthiophene-3-carboxylat (2) anfallende xylolische Lösung direkt mit dem Amin umgesetzt werden ("Eintopfverfahren"). Ein Entfernen des als Katalysator eingesetzten Isocyanats ist für das Gelingen der Umsetzung mit dem Amin nicht zwingend notwendig.

## Patentansprüche

1. Verfahren zum Herstellen von Verbindungen der allgemeinen Formel (I) 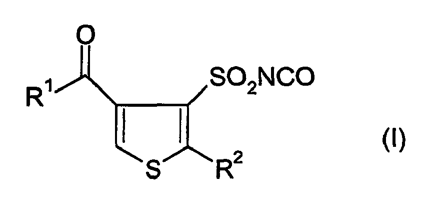 in welcher
R¹ für Alkoxy mit 1 bis 4 Kohlenstoffatomen steht, und
R² für Alkyl mit 1 bis 4 Kohlenstoffatomen steht,
und wobei Verbindungen der Formel (II) 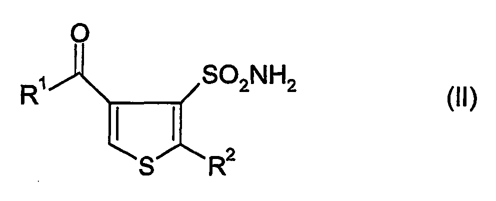 in welcher R¹ und R² die oben angegebene Bedeutung haben,
mit Phosgen in Abwesenheit einer Base und in Gegenwart eines oder mehrerer Verdünnungsmittel sowie gegebenenfalls eines Katalysators umgesetzt werden.

2. Verfahren nach Anspruch 1, **dadurch gekennzeichnet, dass** die Umsetzung bei Temperaturen zwischen 100°C und 150°C durchgeführt wird.

3. Verfahren nach Anspruch 1 oder 2, **dadurch gekennzeichnet, dass** das Verdünnungsmittel aus den Lösungsmitteln Chlorbenzol oder Xylol ausgewählt ist.

4. Verfahren gemäß einem der Ansprüche 1 bis 3, **dadurch gekennzeichnet, dass** die Verbindung der Formel (I) in einem nachfolgenden Reaktionsschritt ohne Zwischenisolierung mit einem Amin umgesetzt wird.

5. Verfahren gemäß Anspruch 4, **dadurch gekennzeichnet, dass** das verwendete Amin 5-Methoxy-4-methyl-2,4-dihydro-3H-1,2,4-triazol-3-on ist.

6. Verfahren nach Anspruch 1, **dadurch gekennzeichnet, dass** das Verfahren in Gegenwart einer katalytisch wirksamen Menge eines Butylisocyanats oder Pentylisocyanats ausgeführt wird.

## Claims

1. A process for preparing compounds of the formula (I) 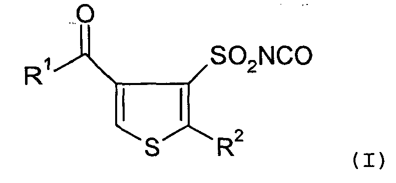 in which
R¹ is alkoxy having from 1 to 4 carbon atoms, and
R² is alkyl having from 1 to 4 carbon atoms,
and in which compounds of the formula (II) 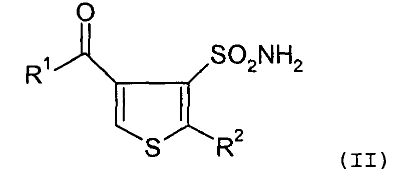 in which R¹ and R² are each as defined above are reacted with phosgene in the absence of a base and in the presence of one or more diluents and also optionally of a catalyst.

2. The process as claimed in claim 1, wherein the reaction is carried out at temperatures between 100°C and 150°C.

3. The process as claimed in claim 1 or 2, wherein the diluent is selected from the solvents chlorobenzene or xylene.

4. The process as claimed in any of claims 1 to 3, wherein the compound of the formula (I) is reacted with an amine in a subsequent reaction step without intermediate isolation.

5. The process as claimed in claim 4, wherein the amine used is 5-methoxy-4-methyl-2,4-dihydro-3H-1,2,4-triazol-3-one.

6. The process as claimed in claim 1, which is performed in the presence of a catalytically active amount of a butyl isocyanate or pentyl isocyanate.

## Revendications

1. Procédé pour la préparation de composés de la formule générale (I) 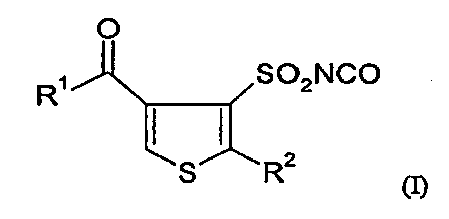 dans laquelle
R¹ représente un groupe alcoxy avec de 1 à 4 atomes de carbone, et
R² représente un groupe alkyle avec de 1 à 4 atomes de carbone,
et dans lequel on fait réagir des composés de la formule (II) 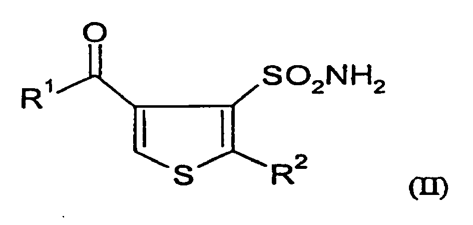 dans laquelle R¹ et R² ont les significations indiquées ci-dessus, avec du phosgène en l'absence d'une base et en présence d'un ou plusieurs agents de dilution ainsi qu'éventuellement d'un catalyseur.

2. Procédé selon la revendication 1, **caractérisé en ce que** la réaction est réalisée à des températures de 100°C à 150°C.

3. Procédé selon la revendication 1 ou 2, **caractérisé en ce que** l'on choisit l'agent de dilution parmi les solvants de chlorobenzène ou de xylène.

4. Procédé selon l'une quelconque des revendications 1 à 3, **caractérisé en ce que** l'on fait réagir le composé de la formule (I) dans une étape de réaction subséquente sans isolement intermédiaire avec une amine.

5. Procédé selon la revendication 4, **caractérisé en ce que** l'amine utilisée est la 5-méthoxy-4-méthyl-2,4-dihydro-3H-1,2,4-triazol-3-one.

6. Procédé selon la revendication 1, **caractérisé en ce que** l'on réalise le procédé en présence d'une quantité catalytiquement active d'un isocyanate de butyle ou d'un isocyanate de pentyle.
